(19) Europäisches Patentamt / European Patent Office / Office européen des brevets

(11) **EP 3 024 479 B2**

(12) **NEW EUROPEAN PATENT SPECIFICATION**
After opposition procedure

(45) Date of publication and mention
of the opposition decision:
**08.02.2023   Bulletin 2023/06**

(45) Mention of the grant of the patent:
**05.06.2019   Bulletin 2019/23**

(21) Application number: **14815008.9**

(22) Date of filing: **15.07.2014**

(51) International Patent Classification (IPC):
**A61K 38/46** (1995.01)          **A61K 38/48** (1995.01)
**C12N 9/94** (1980.01)          **A61P 1/18** (2000.01)

(52) Cooperative Patent Classification (CPC):
**C12N 9/94; A61K 38/465; A61K 38/48;
A61K 38/54; A61P 1/18**

(86) International application number:
**PCT/IB2014/002583**

(87) International publication number:
**WO 2015/019198 (12.02.2015 Gazette 2015/06)**

(54) **METHODS OF PREPARING PANCREATIN**

METHODEN ZUR AUFBEREITUNG VON PANKREATIN

MÉTHODES POUR LE PRÉPARATION DE LA PANCRÉATINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **22.07.2013   US 201361856952 P**

(43) Date of publication of application:
**01.06.2016   Bulletin 2016/22**

(73) Proprietor: **Allergan Pharmaceuticals
International Limited
Dublin 17, D17 E400 (IE)**

(72) Inventors:
- **PIRONTI, Vincenza
  Bray, County Wicklow (IE)**
- **BECKER, Robert
  D-88400 Biberach An Der Riß (DE)**
- **BOLTRI, Luigi
  I-20041 Agrate Brianza (MB) (IT)**
- **GHIDORSI, Luigi
  I-20129 Milano (IT)**
- **ARZUFFI, Paola
  I-24042 Capriate San Gervasio (BG) (IT)**

(74) Representative: **Forresters IP LLP
Skygarden
Erika-Mann-Straße 11
80636 München (DE)**

(56) References cited:
**EP-A2- 0 115 023          CA-A1- 2 419 572
US-A- 3 168 448          US-A1- 2004 057 944
US-A1- 2006 198 838**

- **JI-HWAN HWANG ET AL: "Selective Precipitation of Proteins from Pancreatin Using Designed Antisolvents", INDUSTRIAL & ENGINEERING CHEMISTRY RESEARCH, vol. 46, no. 12, 1 June 2007 (2007-06-01), pages 4289-4294, XP055169811, ISSN: 0888-5885, DOI: 10.1021/ie061280f**
- **SHERMAN ET AL.: "ENZYME PURIFICATION : FURTHER EXPERIMENTS WITH PANCREATIC AMYLASE", J. BIOL. CHEM., vol. 88, 1930, pages 295 - 304**

EP 3 024 479 B2

**Description**

**Field of the Invention**

The present disclosure is directed to high potency pharmaceutical compositions comprising high activity pancreatin (HA-pancreatin) enzymes. The disclosure is also directed to a process of producing HA-pancreatin enzymes and its compositions or dosage forms, and methods for their use. The invention is defined in the claims.

**Background of the Invention**

Exocrine pancreatic insufficiency (EPI), of which the FDA estimates more than 200,000 Americans suffer, involves a physiological disorder wherein individuals are incapable of properly digesting food due to a lack of digestive enzymes made by their pancreas. This loss of digestive enzymes leads to disorders, such as the maldigestion and malabsorption of nutrients, which lead to malnutrition and other consequent and undesirable physiological conditions associated therewith. These disorders are common for those suffering from cystic fibrosis (CF) and other conditions, which compromise the exocrine function of the pancreas, such as pancreatic cancer, pancreatectomy, and pancreatitis. The malnutrition can be life threatening if left untreated, particularly in the case of infants and CF patients. The disorder can lead to impaired growth, a compromised immune response, and shortened life expectancy.

Digestive enzymes, such as pancrelipase enzymes and other pancreatic enzyme products (PEPs) can be administered to at least partially remedy EPI. The administered digestive enzymes allow patients to more effectively digest their food.

The pancrelipase enzymes used for treating EPI are mainly a combination of three enzyme classes: lipase, amylase, and protease, together other enzymes including elastases, phospholipases, and cholesterases, amongst others, and various co-factors and coenzymes with other various co-factors and co-enzymes; the levels or potency in enzyme products are listed. These enzymes are produced naturally in the pancreas and are important in the digestion of fats, proteins and carbohydrates. The enzymes catalyze the hydrolysis of fats into glycerol and fatty acids, starch into dextrin and sugars, and proteins into amino acids and derived substances. Digestion is, however, a complex process involving many other enzymes and substrates that contribute to correct digestive functioning and in producing the full range of digestive products.

Pancrelipase enzymes are typically prepared from porcine pancreatic glands. Other pancrelipase sources include bovine pancreatic glands or pancreatic juices. The natural mammalian source of these enzymes results in a product with an enzyme composition which is similar to that secreted by the human pancreas. Other non-mammalian sources can also be used for example those described in U.S. 6,051,220, U.S. 2004/0057944, 2001/0046493, and WO2006044529.

While the pancrelipase-containing products can offer an effective therapy, there are issues therewith. A need for multiple (4-9) relatively large capsules (high pill load) with every meal decreases a patient's adherence to dosing. Potential microbial and viral contamination consequent to a high pill load is also noted to be undesirable. All of these issues are linked to the enzyme extract being less pure. The purity issue is a consequence of enzyme extraction procedures having been in place for many years and involving the formation of a coarse aqueous blend or slurry, precipitation with alcohol, centrifugation and filtration. Such extraction processes yield final products that may consist of as little as 25% protein. Lipase, an important enzyme in terms of efficacy in these pancrelipase extracts, has an activity is in the region of 100 IU USP/mg. This contrasts with the activity of pure porcine lipase, which has an activity of approximately 25,000 IU/mg (such as pure porcine lipase available from Sigma Aldrich). Using this approximation as a basis for calculation, the products can be estimated to contain less than 0.5% active lipase. Furthermore, the additional consequence of the presence of excess inactive material is that any infectious contamination may be inevitably a part of this bulk and consequently also ingested along with the desirable active components of the mixture. The excess of inactive materials also interfere with techniques aimed at reducing bio-burden, for example through clogging of membrane filters or filtration columns and shielding the extract from ionizing radiation useful for decreasing its potential infectious burden.

A number of pure single enzymes and a mixture of three single enzymes, in one case, have entered clinical development for the treatment of EPI. These are recombinant bile salt stimulated lipase (BSSL) (Exinalda™/Kiobrina®), a recombinant human lipase contained in mothers' milk; Merispase®, a recombinant canine gastric lipase; MS1819, a recombinant lipase; and liprotamase, a mixture consisting of a chemically cross-linked, recombinant bacterial lipase, a protease and an amylase extracted from microbial sources. All of these experimental therapies have so far failed to demonstrate a level of treatment efficacy comparable to that of commercial enzyme extracts from porcine pancreas such as Zenpep® or Ultresa®. Likewise, an FDA advisory board meeting on the 12th January 2011, voted against approval of liprotamase owing to its lower efficacy, in terms of increase in coefficient of fat absorption (CFA) in comparison to that previously obtained with pancrelipase extract products.

There is a clear need for a product which is more concentrated and purified in comparison to existing pancrelipase-containing products, yet which maintains its efficacy for the treatment of EPI, as this would allow better, more convenient and potentially safer products to be produced. There are a number of literature reports that describe the use of pancrelipase as a starting material for the isolation of proteases, lipases or amylases; however, there are no reports of pancrelipase of the type that is found in PEPs or similar products being purified for the purposes of creating an improved product for therapeutic use. In each case, the aim prior to the invention herein described has been to purify a particular enzyme or enzyme fraction over others or to remove certain components without materially increasing overall enzyme activity. In all cases there has also been no direction to produce a HA-pancreatin product for use as a therapeutic agent.

The prior art describing protein purification either aims to extract and isolate a simple protein-rich fraction, as exemplified by pancrelipase, or to separate single proteins or single classes of proteins, e.g., lipases or proteases.

For example, Hwang (Ind. Eng. Chem. Res. 2007, 46, 4289) discloses a relationship between pancreatic enzymes solubility and solvent polarity and reports about the selective precipitation of lipase, protease and amylase from pancreatic proteins. Hwang shows that pancreatin precipitation is enhanced when solvent with reduced polarity is used and that it is maximized when solvent has Hildebrand solubility parameter below 28 $(MPa)^{0.5}$; selective precipitation of amylase and protease increases with decreasing solvent polarity below $34(MPa)^{0.5}$, whereas selective precipitation of lipase is independent by solution polarity, not more than 65% of lipase present in the mixture is recovered. From these results there is no incentive to purify the broad range of pancreatic enzymes together to obtain a HA-pancreatin and there is no incentive for those skilled in the art to preserve a mixture of enzyme classes during purification. The fact that there has been no attempt to purify the pancrelipase which has been used in therapeutic products in well over 60 years is a strong indicator that the benefits of doing this have not been envisaged or appreciated. All attempts to improve the pancrelipase products have focused on single enzymes from non-pancreatic sources, further emphasizing that the use pancrelipase as a source for purer and/or more concentrated enzymes for the manufacture of improved products has not been previously appreciated.

There is no incentive or reason to purify pancrelipase, as is currently used in pharmaceutical and cleaning applications several fold, with the aim of producing a product with a substantially similar qualitative and quantitative profile of enzyme activity. Indeed, the current products fulfill their roles adequately and as such have remained substantially unchanged for over 60 years and there appear to be no descriptions of the markedly improved products or associated preparation processes described herein. Those products containing pure enzymes for the treatment of EPI have all been based on single enzymes or, in one case, a blend of three pure and chemically modified single enzymes, from recombinant technology or microbial sources. There has been no effort to purify a mixture comprising protease, lipase and amylase from the crude pancreas gland extracts that are used for the treatment of EPI, cleaning and tissue digestion. Likewise, there has been no incentive or reports of the enzymes from a pancreatic source being purified individually and then later recombined; such an approach being counter to the aim of achieving an isolated enzyme or enzyme class.

## Summary of the Invention

The present invention is directed to HA-pancreatin enzymes and high potency pharmaceutical compositions or dosage forms thereof. The invention is also directed to high yield process of producing HA-pancreatin and methods for the use of such product.

## Detailed Description of the Invention

The invention is directed to a product (HA (high activity)-pancreatin) that comprises essential enzyme classes having effective and significantly high therapeutic activity, more particularly also having decreased bio-burden of unnecessary biological components and/or undesirable potentially infectious components. The invention is also directed to a process for producing the HA-pancreatin, more particularly in very high yield. The HA-pancreatin would also enable the formulation of smaller and more convenient dosage forms, particularly dosage forms that may be delivered as a single pill, small particle dosage forms for suspension and dosage forms which could be combined with other therapeutic or useful ingredients in a single dosage unit. This innovation would be of particular value to patients suffering from PEI, such as cystic fibrosis patients. The invention would also be useful for formulating into formulations where the age or condition of the patient may require alternative administrative forms than a capsule, e.g., suspension, particles. More concentrated and hence smaller dosage form, unit or particle would be of great value for this patient group. The invention also enables the application of technologies designed to reduce or remove unnecessary or undesirable biological constituents such as microbes and any associated toxins for the reasons outlined above.

The present invention is directed to HA-pancreatin enzymes (HA-pancreatin) and high potency pharmaceutical composition thereof. In a particular embodiment, the HA-pancreatin is porcine derived. The HA-pancreatin comprises lipase, proteases, amylase and has specific lipase activity of at least about 120, or at least about 150, or at least about 200, or at least about 400, or at least about 500 USP IU/mg.

The term "digestive enzyme" used herein denotes an enzyme in the alimentary tract which breaks down the components of food so that they can be taken or absorbed by the organism. Non-limiting examples of digestive enzymes include pancrelipase enzymes (also referred to as pancrelipase or pancreatin), lipase, co-lipase, trypsin, chymotrypsin, chymotrypsin B, pancreatopeptidase, carboxypeptidase A, carboxypeptidase B, glycerol ester hydrolase, phospholipase, sterol ester hydrolase, elastase, kininogenase, ribonuclease, deoxyribonuclease, $\alpha$-amylase, papain, chymopapain, glutenase, bromelain, ficin, $\beta$-amylase, cellulase, $\beta$-galactosidase, lactase, sucrase, isomaltase, and mixtures thereof.

The term "pancreatic enzyme" as used herein refers to any one of the enzyme types present in the pancreatic secretion, such as amylase, lipase, protease, or mixtures thereof, or any extractive of pancreatic origin having enzymatic activity, such as pancreatin.

The terms "pancrelipase enzymes" or "pancrelipase" or "pancreatin" denotes a mixture of several types of enzymes, including amylase, lipase, and protease enzymes. Pancrelipase enzyme is commercially available, for example from Nordmark Arzneimittel GmbH, or Scientific Protein Laboratories LLC.

The term "API" is used herein to denote "digestive enzymes" or "pancrelipase enzymes" or "pancreatin".

The term "lipase" denotes an enzyme that catalyzes hydrolysis of lipids to glycerol and simple fatty acids. Examples of lipases suitable for the present invention include, but are not limited to animal lipase (e.g., porcine lipase), bacterial lipase (e.g., Pseudomonas lipase and/or Burkholderia lipase), fungal lipase, plant lipase, recombinant lipase (e.g., produced via recombinant DNA technology by a suitable host cell, selected from any one of bacteria, yeast, fungi, plant, insect or mammalian host cells in culture, or recombinant lipases, which include an amino acid sequence that is homologous or substantially identical to a naturally occurring sequence, lipases encoded by a nucleic acid that is homologous or substantially identical to a naturally occurring lipase-encoding nucleic acid, etc.), synthetic lipase, chemically-modified lipase, and mixtures thereof. The term "lipids" broadly includes naturally occurring molecules including fats, waxes, sterols, fat- soluble vitamins (such as vitamins A, D, E and K), monoglycerides, diglycerides, triglycerides, phospholipids, etc.

The term "amylase" refers to glycoside hydrolase enzymes that break down starch, for example, $\alpha$-amylases, $\beta$-amylases, $\gamma$-amylases, acid $\alpha$-glucosidases, salivary amylases such as ptyalin, etc. Amylases suitable for use in the present invention include, but are not limited to animal amylases, bacterial amylases, fungal amylases (e.g., Aspergillus amylase, for example, Aspergillus oryzae amylase), plant amylases, recombinant amylases (e.g., produced via recombinant DNA technology by a suitable host cell, selected from any one of bacteria, yeast, fungi, plant, insect or mammalian host cells in culture, or recombinant amylases, which include an amino acid sequence that is homologous or substantially identical to a naturally occurring sequence, amylases encoded by a nucleic acid that is homologous or substantially identical to a naturally occurring amylase-encoding nucleic acid, etc.), chemically modified amylases, and mixtures thereof.

The term "protease" refers generally to enzymes (e.g., proteinases, peptidases, or proteolytic enzymes) that break peptide bonds between amino acids of proteins. Proteases are generally identified by their catalytic type, e.g., aspartic acid peptidases, cysteine (thiol) peptidases, metallopeptidases, serine peptidases, threonine peptidases, alkaline or semi-alkaline proteases, neutral and peptidases of unknown catalytic mechanism. Non-limiting examples of proteases suitable for use in the present invention include serine proteases, threonine proteases, cysteine proteases, aspartic acid proteases (e.g., plasmepsin) metalloproteases and glutamic acid proteases. In addition, proteases suitable for use in the present invention include, but are not limited to animal proteases, bacterial proteases, fungal proteases (e.g., an Aspergillus melleus protease), plant proteases, recombinant proteases (e.g., produced via recombinant DNA technology by a suitable host cell, selected from any one of bacteria, yeast, fungi, plant, insect or mammalian host cells in culture, or recombinant proteases, which include an amino acid sequence that is homologous or substantially identical to a naturally occurring sequence, proteases encoded by a nucleic acid that is homologous or substantially identical to a naturally occurring protease-encoding nucleic acid, etc.), chemically modified proteases, and mixtures thereof.

The pancrelipase enzymes of the composition of present invention can include one or more lipases (i.e., one lipase, or two or more lipases), one or more amylases (i.e., one amylase, or two or more amylases), one or more proteases (i.e., one protease, or two or more proteases), and is mixtures of these enzymes in different combinations and ratios.

Lipase activities in the compositions useful for the present invention can be from about 650 to about 100,000 IU (USP method). It can be from about 675 to about 825 IU, from about 2,500 to about 28,000 IU, from about 2,700 to about 3,300 IU, from about 4,500 to about 5,500 IU, from about 8,000 to about 11,000 IU, from about 13,500 to about 16,500 IU, and from about 18,000 to about 22,000 IU, from about 22,500 to about 27,500 IU, from about 36,000 to about 44,000 IU, and all ranges and sub-ranges there between.

The compositions of the invention preferably contain at least about 650 IU (USP method), at least about 9,000, even more preferably they contain about 20,000, about 40,000, about 60,000, about 80,000, or about 100,000 USP units lipase per dosage unit.

The HA-pancreatin composition according to the present invention may be in powder form or may be in compacted form, e.g., a tablet, or may comprise a plurality of coated and/or uncoated particles. The particles may comprise a core coated with at least one enteric coating, wherein said coating contains an enteric polymer. The above composition besides the coated particles may also comprise uncoated particles of pancrelipase. In particular, the particles are minitablets, microtablets, microparticles, microspheres, microcapsules, micropellets. The particles can have diameters up to about 5 mm. They can have any suitable particle size or shape. For example, the particles can have a particle size range of about 25-5,000 $\mu$m, for example they can be in the form of "minitablets" which have a nominal particle diameter in the range of about 2-5 mm, or they can be "microtablets" which have nominal particle diameters of less than about 2 mm, for example, about 1-2 mm. The particles can have an average particle size of less than about 800 $\mu$m, preferably less than 500 $\mu$m, more preferably less than 200 $\mu$m The particles may have a volume diameter (d(v,0.1)) (defined as the diameter where 10% of the volume distribution is below this value and 90% is above this value) of not less than 400 $\mu$m and a volume diameter d(v,0.9), (defined as the diameter where 90% of the volume distribution is below this value and 10% is above this value) of not more than 800 $\mu$m.

In embodiments where pancrelipase cores are surrounded by an enteric coating the coating acts as a barrier protecting the medication from the acidic environment of the stomach and substantially preventing the release of the medication before it reaches the small intestine. Suitable combinations of enteric coating compositions with other coating compositions can be used to provide the desired type of control over drug release or therapeutic effects. The enteric coating includes at least one enteric polymer and further excipients. The phrase "enteric polymer" means a polymer that protects the digestive enzymes from gastric contents, for example, a polymer that is stable at acidic pH, but can break down rapidly at higher pH or a polymer whose rate of hydration or erosion is slow enough to ensure that contact of gastric contents with the digestive enzymes is relatively minor while it is in the stomach, as opposed to the remainder of the gastro-intestinal tract.

Non-limiting examples of enteric polymers are cellulose acetate phthalate, hydroxypropylmethylcellulose phthalate, hydroxypropylmethylcellulose acetate succinate, polyvinylacetate phthalate, copolymers of methacrylic acid, esters of methylmethacrylate, methylmethacrylate copolymers, and methacrylic acid/ methylmethacrylate copolymers, methacrylic acid- ethyl acrylate copolymer (1:1), shellac, and ethyl cellulose. These polymers are commercially available with different brand names, such as: Cellacefate (cellulose acetate phthalate), Eudragit® L100, S100, L30D, FS30D, L100-55, L30D55 (copolymers of methacrylic acid), Aquateric® (cellulose acetate phthalate), Aqoat® (hydroxypropylmethylcellulose acetate succinate), and HP55® (hydroxypropylmethylcellulose phthalate). The enteric coating may further comprise other excipients such as talc. Preferably, the enteric coating comprises 10-20 wt. % of at least one enteric polymer, wherein each said wt. % is based on the total weight of the coated particles. The coating may further comprise a lipophilic agent, such as a C6 - C30 lipophilic low molecular weight molecule selected from the aliphatic carboxylic acids and alcohols, preferably a C14 - C18 carboxylic acid or alcohol, such as stearic acid, myristic acid, myristic alcohol, or stearyl alcohol. Other optional ingredients of the coating are plasticizer, anti-tacking agents (such as talc, magnesium stearate, colloidal silicon dioxide and combinations thereof; further optionally a low viscosity ethylcellulose). Non-limiting examples of suitable plasticizers include triacetin, tributyl citrate, tri-ethyl citrate, acetyl tri-n-butyl citrate, diethyl phthalate, dibutyl sebacate, polyethylene glycol, polypropylene glycol, castor oil, acetylated mono- and di-glycerides, cetyl alcohol, and mixtures thereof. The preferred plasticizer is a non-phthalate plasticizer or mixtures thereof.

The HA-pancreatin coated or uncoated particles may be prepared according to known processes. For example, the micropellet cores may be prepared by adding a suitable binder to HA-pancreatin followed by extrusion in the presence of a suitable solvent and subsequent spheronization. Controlled spheronization may be applied to generate HA-pancreatin particles with small size. Spray coating, powder layering and fluid bed technologies may be used for preparing beads through coating an inert core. Coacervation processes may also be useful for the preparation of coated pancrelipase particles.

Direct compression may be used to prepare excipient-free compacted tablets. In certain instances the tablet may display gastro-resistance, owing to the in situ formation of a hydrophobic coating layer on contacting gastric fluids.

The compositions comprising the HA-pancreatin may be in any form suited to the dosing of a therapeutic agent containing digesting enzymes, such as for example, they may be in the form of powder, pellets, microspheres, capsules, sachets, tablets, liquid suspensions and liquid solutions.

In one embodiment of the present invention dosage forms that comprise HA-pancreatin, in particular, smaller and/or single dosage forms comprising HA-pancreatin can be prepared. The availability of HA-pancreatin allows to reduce the size of the capsule and/or even to deliver the dose as reduced number of capsules per meal over the typical formulation composition of a 20,000 unit strength Zenpep® size 0 capsule, which is filled with 250-275 mg of API. An adult patient may take from 4 to 10 such capsules per meal. For an overall daily dosage of 200,000 USP U of lipase a patient now takes 10 capsules and the drug product intake is about 2,500-2,750 mg. A purification of at least 2 folds constitutes a meaningful improvement and higher degrees of purification more so. In fact, the HA-pancreatin pharmaceutical dosage form, which takes the form of an orally administered capsule, which may have a content of about 100-110 mg of API (vs 250-275 mg) and therefore for an overall daily dosage of 200,000 USP U of lipase the patient's drug product intake is about 1,000-1,100 mg (vs 2,500-2,750 mg). Furthermore, with the HA-pancreatin capsule size 2 (vs size 0) may be used, thus drastically reducing also the total number of capsules to be administered, or as alternative maintaining a size 0 capsule and properly modulating its content, thus significantly reducing the daily intake. The EPI treatment is a chronic treatment which frequently begins in infancy. The ability to formulate the pancrelipase such that it can be contained in smaller dosage units and/or taken as a reduced number of dosage units per meal constitutes a significant benefit for patients.

The novel dosage forms of the instant invention may also include small particle dosage forms. A pancrelipase product with an increased potency per unit volume would overcome significant issues regarding the reduction of dimension of the beads. The majority of commercial pancrelipase dosage forms are capsules that are filled with pancrelipase beads, which are coated with an enteric polymer. The coating is applied because pancreatic lipase is irreversibly inactivated in acid media. The capsules may be opened and the beads sprinkled on to certain foods and this is an important option for younger patients or those that have difficulty swallowing or coping with the high pill burden. This option does not address the needs of all patients however, as the beads have an appreciable diameter, which may be up to 2 mm. This means the beads cannot be easily suspended in liquids for babies or patients requiring tube feeding. Attempts to reduce the dimensions of the beads result in large increases in total surface area and consequently much more enteric polymer is needed to effectively cover the increased surface area of the particles. This greatly increases the bulk of the dosage form and the amount of polymer ingested to the point where the bulk of dosage form further increases pill burden and the levels of coating excipients may exceed established limits placed on their daily intake.

The availability of a HA-pancreatin, with a much reduced bulk, not only allows the entire dose to be contained in a single dosage unit or in a reduced number of dosage units but also enables the combination of pancrelipase with other compounds. For example, an antacid buffering agent, such as sodium bicarbonate and HA-pancreatin may be combined in a single dosage unit, whereas it would not be possible to contemplate such a combination of pancreatic enzymes and an agent that increases the stomach pH using the current pancrelipase as this would dramatically increase the already very high pill burden as additional capsules/tablets would be required and/or the capsule/tablets would be of excessive size.

The HA-pancreatin also provides the option of providing a dispersible dosage form without an enteric coating as the buffer would prevent acid-inactivation of the lipase component of the pancreatin. In addition, bicarbonate supplementation may also provide therapeutic benefit as bicarbonate secretion is generally reduced in patients with EPI. This novel dosage form can be formulated for immediate or delayed release and be dispersed in a liquid medium. This latter property provides a significant advantage for patients requiring a liquid feed as the components would be readily dispersible in the feed or another convenient medium. These combinations can be delivered in a variety of conventional presentations, such as capsules, tablets, sachets, beads and liquids. As mentioned above, the need to coat the pancrelipase dosage forms with an enteric polymer is a consequence of the instability of the lipase enzyme in acidic media; however, if stomach pH is raised, through the use of proton pump inhibitors, then it has been demonstrated that lipase remains active, presumably because it is not exposed to levels of pH that are low enough to inactivate lipase. This approach is not convenient, nor is it necessarily medically desirable, as it increases pill burden and uses an additional chronic medication to overcome the disadvantages of another. Stomach pH can be temporarily neutralized with simple antacids such as sodium bicarbonate and have been shown to be effective in protecting acid labile drugs, such as the PPI omeprazole, which is a component of the drug Zegerid®. The level of sodium bicarbonate in this drug is 1.1g and the level of omeprazole is either 20 mg or 40 mg and these components are contained in a hard shell capsule.

A single dosage form containing a combination of HA-pancreatin and at least one other active compound, such as $H_2$ antagonist proton pump inhibitors or bile salts, are also disclosed in the present invention.

A product improvement is obtained with the present invention. In fact, the preparation of HA-pancreatin results in a reduction in bioburden simply as a result of the reduction in the amount of material carrying this bioburden. In addition, however, the methodologies used for the preparation process are also able to reduce bioburden and a significantly less encumbered product will be produced as a result. Furthermore, the removal of large quantities of inactive material from the product enables the use of the sterilization techniques that are used for injectable biologies to be applied, e.g., filtration, ultraviolet (UV) light exposure. Again, this represents a significant and unanticipated improvement in product characteristics.

The HA-pancreatin present in the compositions or oral dosage forms of the present invention is prepared according to the following process.

The starting material is pancreatin. In the present invention, we may refer to it also by using the terms "API", or "starting pancreatin", or "starting pancreatic enzymes", or "native pancreatin", "starting pancrelipase", or "native pancrelipase".

A convenient starting material is porcine derived pancrelipase as commercially available for example from Nordmark Arzneimittel GmbH, or Scientific Protein Laboratories LLC. Similar extracts from bovine or other mammalian sources may also be used. The preferred starting material is porcine derived pancrelipase. The extraction procedures used to produce the crude extract can be summarized as comprising the following steps: pig glands ground wet; addition of a pancrelipase 'activator'; treatment of the "crude enzyme slurry" with cold and hot isopropanol to precipitate proteins and remove lipids; centrifugation and filtration steps to remove fibrous and to compact and concentrate; vacuum drying of "wet cake"; de-lumped and milling of the "wet cake" for bulk density and particle size. This dry product is the pancreatin used in current products.

The HA-pancreatin of the invention is prepared by further treating of the starting pancreatin; it preserves those elements that are key to the efficacy of pancreatic enzyme based products and removes those elements which are non-essential.

The material resultant from the process of the invention is the HA-pancreatin.

The HA-pancreatin having specific lipase activity of at least about 120 USP IU/mg is prepared using a process comprising treating pancreatin with a solvent, wherein said solvent has Hildebrand solubility parameter (SP) comprised between 28 and 45 $(MPa)^{0.5}$, and said solvent is one organic solvent or a mixture of organic solvents or a mixture of at least one organic solvent and aqueous solvent and the process is carried out at about 4°C.

In one embodiment the HA-pancreatin having specific lipase activity of at least about 120 USP IU/mg is prepared using a process comprising treating pancreatin with a solvent, wherein said solvent has Hildebrand solubility parameter (SP) comprised between 28 and 38 $(MPa)^{0.5}$, and said solvent is one organic solvent or a mixture of organic solvents or a mixture of at least one organic solvent and aqueous solvent and the process is carried out at about 4°C.

In one specific embodiment the HA-pancreatin having specific lipase activity of at least about 120 USP IU/mg is prepared using a process comprising treating pancreatin with a solvent, wherein said solvent has Hildebrand solubility parameter (SP) comprised between 28 and 34 $(MPa)^{0.5}$, and said solvent is one organic solvent or a mixture of organic solvents or a mixture of at least one organic solvent and aqueous solvent and the process is carried out at about 4°C.

In another specific embodiment the HA-pancreatin having specific lipase activity of at least about 120 USP IU/mg is prepared using a process comprising treating pancreatin with a solvent, wherein said solvent has Hildebrand solubility parameter (SP) comprised between 34 and 38 $(MPa)^{0.5}$, and said solvent is one organic solvent or a mixture of organic solvents or a mixture of at least one organic solvent and aqueous solvent and the process is carried out at about 4°C.

In another embodiment the HA-pancreatin having specific lipase activity of at least about 120 USP IU/mg is prepared using a process comprising treating pancreatin with a solvent, wherein said solvent has Hildebrand solubility parameter (SP) comprised between 34 and 45 $(MPa)^{0.5}$, and said solvent is one organic solvent or a mixture of organic solvents or a mixture of at least one organic solvent and aqueous solvent and the process is carried out at about 4°C.

In one specific embodiment the HA-pancreatin having specific lipase activity of at least about 120 USP IU/mg is prepared using a process comprising treating pancreatin with a solvent, wherein said solvent has Hildebrand solubility parameter (SP) comprised between 38 and 45 $(MPa)^{0.5}$, and said solvent is one organic solvent or a mixture of organic solvents or a mixture of at least one organic solvent and aqueous solvent and the process is carried out at about 4°C.

The HA-pancreatin may have specific lipase activity of at least about 120, or at least about 150, or at least about 200, or at least about 400, or at least about 500 USP IU/mg.

The Hildebrand solubility parameter is a numerical value that indicates the relative solvency behavior of a specific solvent. It is derived from the cohesive energy density of the solvent, which in turn is derived from the heat of vaporization. Hildebrand values are available from literature sources, such as from Barton Handbook of Solubility Parameters, CRC Press, 1983. The process solvent is one organic solvent or a mixture of more organic solvents or a mixture of at least one organic solvent and aqueous solvent; the mixture of organic solvent and aqueous solvent may comprise one or more organic solvent and one or more aqueous solvent. The solvent may have the following solubility values: 45, 42, 40, 38, 36, 35, 34, and 28.

The organic solvent may be chosen from the group of solvent comprising n-pentane, n-hexane, n-heptane, diethylether, cyclohexane, carbon tetrachloride, ethyl acetate, tetrahydrofuran, chloroform, trichloroethylene, acetone, dimethylformamide, n-propanol, isopropanol, ethanol, dimethylsulfoxide butylalcohol, methanol, acetonitrile, dioxane, and methylenchloride. Preferred organic solvents are acetone, isopropanol, and ethanol.

The aqueous solvent may be chosen from the group consisting of: water, or buffer solutions. Preferred buffers have pH=7 or pH=4, they may be respectively pH=7: 10 mM phosphate buffer and pH=4.0: 10 mM acetate buffer.

In one embodiment of the invention the solvent is a mixture comprising one or more organic solvent and one aqueous solvent, said mixture has Hildebrand solubility parameter ranging from 28 to 45 $(MPa)^{0.5}$. In embodiment of the invention the solvent is a mixture comprising one or more organic solvent and one aqueous solvent, said mixture has Hildebrand solubility parameter ranging from 28 to 38 $(MPa)^{0.5}$. In one specific embodiment the solvent is a mixture comprising one or more organic solvent and one aqueous solvent, said mixture has Hildebrand solubility parameter ranging from 28 to 34 $(MPa)^{0.5}$. In one specific embodiment the solvent is a mixture comprising one or more organic solvent and one aqueous solvent, said mixture has Hildebrand solubility parameter ranging from 34 to 38 $(MPa)^{0.5}$. In another embodiment the solvent is a mixture comprising one or more organic solvent and one aqueous solvent, said mixture has Hildebrand solubility parameter ranging from 34 to 45 $(MPa)^{0.5}$. In one specific embodiment the solvent is a mixture comprising one or more organic solvent and one aqueous solvent, said mixture has Hildebrand solubility parameter ranging from 38 to 45 $(MPa)^{0.5}$. SP of solvent mixture is calculated using the Hildebrand solubility parameters.

In one embodiment the solvent has SP of 38 $(MPa)^{0.5}$ and is a mixture of organic solvent with aqueous solvent.

Few examples of such binary solvent having SP= 38 are:

acetone- buffer: volumetric ratio of acetone to pH=7 buffer is 35:65; volumetric ratio of acetone to pH=4.0 buffer is 35:65; where: SP(acetone)=20.2, SP(buffer) =47.9;

ethanol- buffer: volumetric ratio ethanol to pH=7 buffer is 45:55; volumetric ratio of ethanol to pH=4.0 buffer is 45:55; where SP(ethanol)= 26.0, SP(buffer) =47.9;

In another embodiment a binary solvent with SP =34 (MPa)$^{0.5}$ is: acetone-buffer: volumetric ratio of acetone to pH=7 buffer is 50:50; where SP(acetone)=20.2, SP(buffer) =47.9.

In yet another embodiment a binary solvent with SP =35 (MPa)$^{0.5}$ is acetone-buffer: volumetric ratio of acetone to pH=7 buffer is 45: 55; where SP(acetone)=20.2, SP(buffer) =47.9.

In one embodiment of the present invention (single-step process), the treating of pancreatin with a solvent having SP of 28-45 (MPa)$^{0.5}$ comprises the following steps: a1) suspending pancreatin in the solvent under stirring; a2) separating at the insoluble portion (pellet) from the soluble portion (supernatant) of the mixture of step a2; a3) drying the insoluble portion obtained in step a1; and wherein the steps a1-a3 are carried out at temperature below room temperature. Suitable temperature for carrying out the process is 4°C.

In one embodiment of the present invention (single-step process), the treating of pancreatin with a solvent having SP of 34-38 (MPa)$^{0.5}$, comprises the following steps: a1) suspending pancreatin in the solvent under stirring; a2) separating at the insoluble portion (pellet) from the soluble portion (supernatant) of the mixture of step a2; a3) drying the insoluble portion obtained in step a1; and wherein the steps al-a3 are carried out at about 4°C.

Step 1a is preferably carried out for about 60 minutes; the preferred temperature is 4°C. Separation step (step a2) may be carried out by different methods such as centrifugation, sedimentation or filtration. Drying step (a3) can be carried out for example in a high efficiency dryer, vacuum pump, or freeze dryer; other methods can also be used. The concentration of pancreatin in solvent in step a1 is preferably in amount comprised between 0.050 and 0.3 mg/mL, preferably between 0.065 and 0.1 mg/mL, it is preferably 0.065 or 0.1 mg/mL.

In one specific embodiment of the single-step process the solvent has SP of 38 (MPa)$^{0.5}$ and it is a mixture of acetone and pH 7 buffer (such as 10 mM phosphate), and the pancreatin in step a1 is in concentration of 10 g/mL.

In another embodiment of the present invention (two-steps process), where the solvent is a mixture of organic solvent and aqueous solvent, the pancreatin is first dispersed in the aqueous solvent and then the organic solvent is added thereon. In this embodiment the step a1 comprises the following steps: a1.1 (suspension) suspending pancreatin in the aqueous solvent under stirring; a1.2 (precipitation) adding to the suspension of step 1a the organic solvent or mixture thereof. The mixture of step a1.1 is kept under static condition. Duration of time of step a1.1 is from about 10 to about 30 minutes depending upon the scale and the equipment; duration of time of step a1.2 is about 30 minutes.

The pancreatin in aqueous solvent is preferably in amount comprised between 0.050 and 0.3 mg/mL, preferably between 0.1 and 0.3 mg/mL, preferably 0.1 or 0.3 mg/mL, the organic solvent is preferably either ethanol or acetone and the aqueous solvent is preferably a pH=4.0 buffer (such as 10mM acetate buffer) or a pH 7 buffer (such as 10 mM phosphate).

In one specific embodiment of the single-step process the solvent has SP of 38 (MPa)$^{0.5}$ and it is a mixture of acetone and pH 7 buffer (such as 10 mM phosphate), and the pancreatin in step a1 is in concentration of 0.1 mg/mL.

In another specific embodiment of the single-step process the solvent has SP of 38 (MPa)$^{0.5}$ and it is a mixture of acetone and pH 4 buffer (such as 10 mM acetate buffer), and the pancreatin in step a1 is in concentration of 0.1 mg/mL.

In yet another embodiment of the invention (multi-step process), when the solvent is a mixture of organic solvent and aqueous solvent, the pancrelipase is first dispersed in the aqueous solvent and then the organic solvent is added to the soluble portion of this aqueous dispersion. In this embodiment the process step a1 comprises the following steps: a1.1) (suspension) suspending pancreatin in the aqueous solvent under stirring; a1.2) (separation) separating the soluble portion (supernatant) of step a1.1 from the insoluble portion (pellet); a1.3) (precipitation) adding to the soluble portion of step a1.2 the organic solvent or mixture thereof.

Step a1.1 is preferably carried out for about 30 minutes; mixture of step a1.3 is kept under static condition for about 15 minutes; a preferred temperature for these steps is 4°C.

The pancreatin in aqueous solvent is preferably in amount comprised between 0.05 and 0.3 mg/mL, preferably from 0.1 to 0.3 mg/mL, preferably 0.1 or 0.3 mg/mL. The SP of the solvent used is preferably 38. The organic solvent is preferably either ethanol or acetone and the aqueous solvent is preferably a pH=4.0 buffer (such as 10mM acetate buffer) or a pH 7 buffer (such as 10 mM phosphate).

In one specific embodiment of the multi-step process the solvent has SP of 38 (MPa)$^{0.5}$ and it is a mixture of acetone and pH 4.0 buffer (such as 10mM acetate buffer), and the pancreatin in step a1 is in concentration of 0.3 mg/mL.

In yet another embodiment of the multi-step process the solvent has SP of 38 (MPa)$^{0.5}$ and it is a mixture of ethanol and pH 4.0 buffer (such as 10mM acetate buffer), and the pancreatin in step a1 is in concentration of 0.3 mg/mL.

In yet another embodiment of the multi-step process the solvent has SP of 38 (MPa)$^{0.5}$ and it is a mixture of ethanol and pH 4.0 buffer (such as 10mM acetate buffer), and the pancreatin in step a1 is in concentration of 0.1 mg/mL.

In yet another embodiment of the multi-step process the solvent has SP of 38 (MPa)$^{0.5}$ and it is a mixture of acetone and pH 7 buffer (such as 10 mM phosphate) and the pancreatin in step 1a is in concentration of 0.3 mg/mL.

Separation steps (step a2 and a1.2) may be carried out by different methods such as centrifugation or filtration. All process steps of the HA-pancreatin preparation process are carried out under temperature control, which is about 4°C; humidity may be also controlled.

The process of the invention may also include sterilization and viral inactivation or viral load reduction of the HA-pancreatin, which may be carried out for example, by filtration, heating, irradiation (ultraviolet radiation, X-radiation, beta-radiation and gamma-radiation), high pressure treatment and/or alkylation of nucleic acids such as using beta-propriolactone (BPL). Heating at temperature such as above 85°C, preferably between 85°C and 100°C for suitable period of time, such as above 18 hours and preferably between 18 and 48 hours, even more preferably between 18 and 30 hours is also effective in reducing the viral contaminants. Heating at lower temperature (84°C, preferable 80°C) may be carried out on solid HA-pancreatin with residual moisture of 0.5 weight % or less.

It is clear from the above that there are many advantages to the approach disclosed in the present invention. It preserves the natural spectrum and the natural source of digestive enzymes that are present in digestive juices and maintain the digestion behavior of the starting material. It removes those materials that are not required for digestion that remain as a result of the crude extraction process, thus decreasing the bulk of the API. It enables the reduction or removal of bacterial or viral contaminants. It enables a control of the ratios of the mixed enzyme classes to one another. Moreover, it enables the API to be formulated in a number of dosage forms with high potency.

The invention described herein results in a product which is superior to present products and a represents a marked advance in therapy for the reasons outlined above.

**Experimental**

Materials

*Pancrelipase* (API, starting pancrelipase or pancreatin, native pancrelipase or pancreatin) is provided by Nordmark. It is extracted from porcine pancreas and typically contains about 30% of proteins (quantified with Bradford method) and has a lipase activity of 94.4 IU/mg, protease activity of 253.2 IU/mg, amylase activity of 420.3 IU/mg; P/L ratio is 2.7, A/L ratio is 4.5; water content is 0.3. This material is analyzed by mono and bi-dimensional electrophoresis followed by Maldi Tof characterization in order to identify pancrelipase constituents (Mario Negri Institute, Milan, Italy). Bidimensional electrophoresis on this extract shows there to be at least 50 protein/ peptide spots in a fraction that is apparently readily soluble in water and 30 protein/ peptide spots in a fraction which is apparently less readily soluble in water. Solubility determinations are confused by the fact that active enzymes may be adsorbed onto or otherwise trapped by water-insoluble components of the pancrelipase. Solubility will also be a function of pH. The spots, corresponding to individual proteins in the mixture, and images of gels prepared using the more soluble and the less soluble fractions are analyzed after cutting out and digesting the major spots with bovine trypsin and analysing using matrix-assisted laser desorption/ ionization-time of flight mass spectrometry (MALDI-TOF) and zymography. The peptide mass fingerprints are compared with those from library references and amylase, lipase, colipase, carboxypeptidase A1 and B, elastase 2A and 1, chymotrypsin, trypsin, and phospholipase A2 are identified. The extract is clearly both relatively crude and contains many extraneous proteins in addition to a number of active enzyme species.

*Enteral formula:* Peptamen® Junior 1.0 Cal (Nestlé, package of 250 mL): fat content: 3.8 g/100 mL, protein content: 3 g/100mL, fat and carbohydrate content: 20.4 g/Ml.

Methods

*Lipolytic activity:* Measurement is carried out with a method based on the compendial procedure of lipase assay described in the pancrelipase enzymes USP monograph, which is based on the titration, by means of pH-stat method, of the free fatty acids formed from the hydrolysis of esterified fatty acids in the substrate used (olive oil). It is based on the following principle: lipase catalyses the hydrolysis of the triglycerides, which leads to the formation of free fatty acids (FFA). The titration of the formed FFA according to time provides for the determination of the enzymatic activity of lipase, which can be expressed in units: 1U = 1 μmole of formed FFA per minute. The reaction occurs by maintaining a steady pH value through an experimental system that provides for the addition of NaOH (titrant) when the pH value changes compared to a fixed value (pHstat method). The quantity of added titrant according to time corresponds to the quantity of FFA formed by the lipase action on the triglycerides. The curve slope {added titrant = f (volume (mL)/ time (minutes))} gives the lipase enzymatic activity.

The lipase activity (LA) reported hereunder is always expressed as IU USP.

The lipase specific activity (LSA) reported hereunder is always expressed as IU USP/mg.

*Proteolytic and amilolytic activity:* Measurements are carried out according to the compendial procedure described in the pancrelipase enzymes USP monograph. Specific enzymatic activity (SA) reported hereunder is always expressed as IU USP/mg.

*Water content* is measured by TGA at 80°C for 4 hours (samples 18, 19 and 20) or with Karl Fisher method (samples 26, 27 and 28).

*Triglycerides* are extracted with hexane: isopropanol (3:2) using cholesteryl palmitate as internal standard and analyzed by HPLC; peaks are identified by comparing all the retention times with a standard triolein solution.

*Protein analysis:* Total Protein Content is quantified with a Bradford Assay.

*Carbohydrate analysis:* 1) Short chain sugars are analyzed by HPLC using xylitol as internal standard; peaks are identified by comparing all the retention times with sugars standards i.e., maltose. 2) Maltodextrins is extracted in presence of Carrez I and Carrez II and analyzed by HPLC; peaks are identified by comparing all the retention times with maltodextrins standards i.e., maltose monohydrate, maltotriose, maltotetraose, maltopentaose, maltohexaose and maltoheptaose.

## Examples

**Example 1** Preparation of HA-pancreatin; 0.1 g/mL; multi-steps: suspension, separation, precipitation; (SP= 38: acetone: aqueous solvent = 35: 65; ethanol: solvent aqueous solvent = 45: 55)

Step a1.1- Suspension: starting pancrelipase is dispersed in aqueous solvent at concentration of 0.1 g/mL at 4°C and kept under stirring for 30 minutes. The experiments are carried out at laboratory scale using either 650 mg (when organic solvent is acetone) or 550 mg (when organic solvent is ethanol) of starting pancrelipase. Four different aqueous solvents are tested for pancrelipase suspension: 1) pH=4.0 buffer (10mM acetate buffer); 2) pH=7.0 buffer (10 mM phosphate); 3) deionized water (DW); 4) pH=4.0 buffer (10mM acetate) with NaCl (0.5M).

Step a1.2- Separation: the suspension from step a1.1 is centrifuged (10 minutes, 4°C, about 11,000 g) and the supernatant (SN) is separated from the pellet.

Step a1.3- Precipitation: the organic solvent is added to the supernatant of step a1.2 and the mixture is kept at 4°C for 15 minutes in static condition. The organic solvent is either acetone or ethanol. Acetone is added in amount of 35 parts (volume) per each 65 parts (volume) of aqueous solvent; ethanol is added in amount of 45 parts (volume) per each 55 parts (volumes) of aqueous solvent.

Step a2- Separation: the mixture is centrifuged (10 minutes, 4°C, about 11,000 g) to separate supernatant from pellet, which contains the pancrelipase enzymes. The pellet is re-suspended in the aqueous solvent used in step a1.1 (LA in pellets after re-suspension, precipitation).

The materials of the different steps are analyzed. The amount of pancrelipase, expressed as lipase activity (LA) is measured:

- in suspension of step a1 (LA in suspension; LA-Sa1.1);

- in supernatant obtained in step a2 (LA in SN, precipitation, LA-SN);

- in pellets obtained in step a2 and then re-suspended in the starting medium (LA in pellets, precipitation, LA-P).

Results are reported in Tables 1, 2, 3, and 4.

Table 1

| Sample | Experimental conditions | | Suspension (Step a1.1) | Separation (Step a2) | | | | Yield |
|---|---|---|---|---|---|---|---|---|
| | Medium | Solvent | LA-Sa1.1 | Supernatant (SN) | | Pellet (P) | | |
| | | | | LA-SN | % LA-SN/LA-Sa1.1 | LA-P | % LA-P/LA-Sa1.1 | |
| 1 | pH 4.0 | A | 33528.2 | 5820.0 | 17.4 | 25376.4 | 75.7 | 93.1 |
| 2 | Water | A | 31974.0 | 8105.5 | 25.4 | 22659.2 | 70.9 | 96.3 |
| 3 | pH 4.0 | E | 35388.9 | 3464.3 | 9.8 | 27417.6 | 77.5 | 87.3 |
| 4 | Water | E | 30369.8 | 4301.0 | 14.2 | 20835.1 | 68.6 | 82.8 |
| 5 | pH 4.0+NaCl | A | 47761.0 | 9408.0 | 19.7 | 34543.3 | 72.3 | 92.0 |
| 6 | pH 4.0+NaCl | E | 40364.0 | 10410.4 | 25.8 | 27101.5 | 67.1 | 92.9 |
| 7 | pH 7.0 | A | 40388.9 | 9798.8 | 24.3 | 30178.8 | 74.7 | 99.0 |
| LA=lipase activity (IU USP); A= acetone. E=ethanol; S=suspension; SN=supernatant; P=pellet | | | | | | | | |

Table 1 shows that precipitation of pancrelipase (step a1.3) allows for a good recovery of lipase activity in the precipitate portion (pellet), which ranges from about 67 to about 77 %. Yield is the % of total lipase activity of step a2 (pellet and supernatant) with respect to the lipase activity of the initial suspended pancrelipase, expressed as lipase activity in the suspension of step a1.1 (Sa1.1): (LA-SN +LA-P)/(LA-Sa1.1).

Table 2

| Sample | Experimental conditions | | Theor. LA in Step a1.1 | Separation (Step a2) | | | | Yield |
|---|---|---|---|---|---|---|---|---|
| | Medium | Solvent | | Supernatant (SN) | | Pellet (P) | | |
| | | | | LA-SN | % LA-SN/theor LA-Sa1.1 | LA-P | % LA-P/theor LA-Sa1.1 | |
| 1 | pH 4.0 | A | 61605.4 | 5820.0 | 9.4 | 25376.4 | 41.2 | 50.6 |
| 2 | Water | A | 61458.2 | 8105.5 | 13.2 | 22659.2 | 36.9 | 50.1 |
| 3 | pH 4.0 | E | 51940.8 | 3464.3 | 6.7 | 27417.6 | 52.8 | 59.5 |
| 4 | Water | E | 51894.0 | 4301.0 | 8.3 | 20835.1 | 40.1 | 48.4 |
| 5 | pH 4.0+NaCl | A | 61452.0 | 9408.0 | 15.3 | 34543.3 | 56.2 | 71.5 |
| 6 | pH 4.0+NaCl | E | 52031.5 | 10410.4 | 20.0 | 27101.5 | 52.1 | 72.1 |
| 7 | pH 7.0 | A | 61470.4 | 9798.8 | 15.9 | 30178.8 | 49.1 | 65.0 |
| LA=lipase activity (IU USP); A= acetone. E=ethanol; S=suspension; SN=supernatant; P=pellet; Theor.=theoretical. | | | | | | | | |

The process yield ranges from about 37 to about 56%. Yield % is the total lipase activity in the pellet and in supernatant of step a2 with respect to theoretical lipase activity of pancrelipase used in step a1.1, which is calculated by factoring specific activity of untreated raw material (i.e., 94.4 IU USP/mg as determined according to compendial USP method) and its initial weight.

Table 3

| Sample | Experimental conditions | | Separation (Step a2) | | EF |
| | Medium | Solvent | Supernatant (SN) | Pellet (P) | |
| | | | LSA | LSA | |
| 1 | pH 4.0 | A | 10.7 | 239.4 | 2.5 |
| 2 | Water | A | 14.5 | 233.6 | 2.5 |
| 3 | pH 4.0 | E | 10.1 | 179.2 | 1.9 |
| 4 | Water | E | 11.5 | 145.7 | 1.5 |
| 5 | pH 4.0+ NaCl | A | 14.7 | 101.3 | 1.1 |
| 6 | pH 4.0+ NaCl | E | 18.2 | 80.9 | 0.9 |
| 7 | pH 7.0 | A | 18.7 | 181.8 | 1.9 |

LSA= lipase specific activity (IU USP/mg); A= acetone. E=ethanol; SN=supernatant; EF= enrichment factor= LSA in pellet of step a2/ theoretical lipase specific activity in step 1.1a i.e. 94.4 IU USP/mg as determined according to compendial USP method.

The enrichment factor (pH independent) is determined by calculating the ratio between the lipase activity in the pellet of step a2 and the lipase activity of the starting pancreatin (which is 94.4 IU/mg). Enrichment factor up to 2.5 is obtained with this process. The enrichment is confirmed also by HPLC profile analysis.

With regards to the other digestive enzymes, the amylase content in the final pellet (precipitate of step a2) is lower than the amylase content in the starting pancrelipase.

**Example 2** Preparation of HA-pancreatin; 0.3 g/mL; multi-steps: suspension, separation, precipitation (SP= 38: acetone: aqueous solvent = 35: 65; ethanol: aqueous solvent = 45: 55)

Step a1.1- Suspension: pancrelipase (API) is dispersed in aqueous solvent at concentration of 0.3 g/mL at 4°C and stirred for 30 minutes. The experiments are carried out at laboratory scale using either 650 mg (when organic solvent is acetone) or 550 mg (when organic solvent is ethanol) of starting pancrelipase. Two experiments are run each in a different aqueous solvent: 1) pH=4.0 buffer (10mM acetate buffer); 2) pH=7.0 buffer (10 mM phosphate).

Step a1.2- Separation: the suspension from step a1 is centrifuged (10 minutes, 4°C, about 11,000 g) and the supernatant (SN) is separated from the pellet.

Step a1.3- Precipitation: the organic solvent is added to the supernatant of step a1.2 and the mixture is kept at 4°C for 15 minutes in static condition. The organic solvent is either acetone or ethanol. Acetone is added in amount of 35 parts (volume) per each 65 parts (volume) of aqueous solvent. Ethanol is added in amount of 45 parts (volume) per each 55 parts (volumes) of aqueous solvent.

Step a2- Separation: the mixture is centrifuged (10 minutes, 4°C, about 11,000 g) to separate supernatant from pellet, which contains the pancrelipase enzymes. The pellet is re-suspended in the starting aqueous solvent (LA in pellets after re-suspension, precipitation).

Step a3- Drying: the pellet of step a2 is dried.

The materials of the different steps are analyzed. The amount of pancrelipase is expressed as lipase activity (LA) and it is measured:

-    in suspension of step a1.1 (LA in suspension; LA-Sa1.1);

-    in supernatant obtained in step a2 (LA in SN, precipitation, LA-SN);

- in pellets obtained in step a2 and then re-suspended in the starting aqueous medium (LA in pellets, precipitation, LA-P).

Results are reported in Tables 4, 5, and 6.

Table 4

| Sample | Experimental conditions | | Suspension (Step a1.1) | Separation (Step a2) | | | | |
|---|---|---|---|---|---|---|---|---|
| | | | | Supernatant (SN) | | Pellet (P) | | |
| | Medium | Solvent | LA (Sa1.1) | LA-SN | % LA-SN/LA-Sa1.1 | LA-P | % of LA-P/LA-Sa1.1 | Yield |
| 8 | pH 4.0 | A | 139968.7 | 11856.6 | 8.5 | 112830.4 | 80.6 | 89.1 |
| 9 | pH 4.0 | E | 107348.7 | 5035.2 | 4.7 | 99146.6 | 92.4 | 97.0 |
| 10 | pH 4.0 | A | 137050.4 | 8778.9 | 6.4 | 108734.7 | 79.3 | 85.7 |
| 11 | pH 4.0 | E | 115965.7 | 5097.9 | 4.4 | 88019.7 | 75.9 | 80.3 |
| 12 | pH 7.0 | A | 137267.4 | 10535.3 | 7.7 | 108038.6 | 78.7 | 86.4 |
| 13 | pH 7.0 | E | 118929.3 | 11340.9 | 9.5 | 86636.2 | 72.8 | 82.4 |
| LA=lipase activity (IU USP);; A= acetone. E=ethanol; S=suspension; SN=supernatant; P=pellet | | | | | | | | |

Table 4 shows that recovery after precipitation is very good (73-92%).

Table 5

| Sample | Experimental conditions | | | Separation (Step a2) | | | | Yield |
|---|---|---|---|---|---|---|---|---|
| | | | | Supernatant (SN) | | Pellet (P) | | |
| | Medium | Solvent | Theor. LA in Step a1.1 | LA | %LA-SN/theor LA-Sa1.1 | LA | % LA-P/theor LA-Sa1.1 | |
| 8 | pH 4.0 | A | 184080.0 | 11856.6 | 6.4 | 112830.4 | 61.3 | 67.7 |
| 90 | pH 4.0 | E | 155760.0 | 5035.2 | 3.2 | 99146.6 | 63.7 | 66.9 |
| 10 | pH 4.0 | A | 184080.0 | 8778.9 | 4.8 | 108734.7 | 59.1 | 63.8 |
| 11 | pH 4.0 | E | 155760.0 | 5097.9 | 3.3 | 88019.7 | 56.5 | 59.8 |
| 12 | pH 7.0 | A | 184080.0 | 10535.3 | 5.7 | 108038.6 | 58.7 | 64.4 |
| 13 | pH 7.0 | E | 155760.0 | 11340.9 | 7.3 | 86636.2 | 55.6 | 62.9 |
| LA=lipase activity (IU USP);; A= acetone. E=ethanol; S=suspension; SN=supernatant; P=pellet; Theor. =theoretical. | | | | | | | | |

The overall yield of multi-steps process carried out on 0.3g/mL pancrelipase concentration ranges from about 56 to about 64.

Table 6

| Sample | Experimental conditions | | Separation (Step a2) | | EF |
|---|---|---|---|---|---|
| | Medium | Solvent | Supernatant (SN) | Pellet (P) | |
| | | | LSA | LSA | |
| 8 | pH 4.0 | A | 6.2 | 197.9 | 2.1 |
| 9 | pH 4.0 | E | 5.3 | 250.4 | 2.7 |
| 10 | pH 4.0 | A | 7.5 | 284.6 | 3.0 |

(continued)

| Sample | Experimental conditions | | Separation (Step a2) | | EF |
|---|---|---|---|---|---|
| | Medium | Solvent | Supernatant (SN) | Pellet (P) | |
| | | | LSA | LSA | |
| 11 | pH 4.0 | E | 5.5 | 185.7 | 2.0 |
| 12 | pH 7.0 | A | 8.4 | 248.9 | 2.6 |
| 13 | pH 7.0 | E | 11.1 | 180.9 | 1.9 |
| LSA= lipase specific activity (IU USP/mg); A= acetone. E=ethanol; SN=supernatant; EF= enrichment factor= LSA in pellet step a2 (IU USP/mg)/ theoretical lipase activity in step a1.1 (IU USP/mg). | | | | | |

The enrichment factor is determined by calculating the ratio between the lipase activity in the pellet of step a2 and the lipase activity of the starting pancrelipase of step a1.1 (which is 94.4 U/mg). Enrichment factor ranges from 1.9 to 3.0.

**Example 3** Preparation of HA-pancreatin; 0.1 g/mL; two-steps: suspension, precipitation (SP= 38: acetone: aqueous solvent = 35: 65; ethanol: aqueous solvent = 45: 55, where SP (acetone) =20.2, SP (ethanol) = 26.0, SP (buffer) =47.9)

Step a1.1- Suspension: pancrelipase is dispersed in aqueous solvent at concentration of 0.1 g/mL at 4°C and kept under stirring for 30 minutes. The experiments are carried out at laboratory scale using either 650 mg (when organic solvent is acetone) or 550 mg (when organic solvent is ethanol) of starting pancrelipase. Two experiments are run each in a different aqueous solvent: 1) pH=4.0, 10 mM acetate buffer; 2) pH=7.0, 10 mM phosphate buffer.

Step a1.2- Precipitation: the organic solvent is added to the suspension of step a1.1 and this mixture is kept at 4°C for 15 minutes. The organic solvent is either acetone or ethanol. Acetone is added in amount of 35 parts (volume) per each 65 parts (volume) of aqueous solvent. Ethanol is added in amount of 45 parts (volume) per each 55 parts (volumes) of aqueous solvent.

Step a2- Separation: the mixture is centrifuged (10 min, 4°C, about 11,000 g) to separate supernatant from pellet, which contains the pancreatic enzymes. The pellet is re-suspended in the starting aqueous solvent (LA in pellets after re-suspension, separation).

The materials of the different steps are analyzed. The amount of pancrelipase, expressed as lipase activity, is measured along the whole process:

- in suspension of step a1.1 (LSA in suspension; LA-Sa1.1);

- in supernatant obtained in step a2 (LA in SN, separation, LA-SN);

- in pellets obtained in step a2 and then re-suspended in the starting medium (LA in pellets, precipitation, LA-P).

Results are reported in Tables 7, 8, and 9.

Table 7

| Sample | Experimental conditions | | Suspension (Step a1.1) | Separation (Step a2) | | | | Yield |
|---|---|---|---|---|---|---|---|---|
| | | | | Supernatant (SN) | | Pellet (P) | | |
| | Medium | Solvent | LA (Sa1.1) | LA | % LA-SN/LA-Sa1.1 | LA | % LA-P/LA-Sa1.1 | |
| 14 | pH 4.0 | A | 48960.7 | 4688.0 | 9.6 | 45021.4 | 92.0 | 101.5 |

(continued)

| Sample | Experimental conditions | | Suspension (Step a1.1) | Separation (Step a2) | | | | Yield |
|---|---|---|---|---|---|---|---|---|
| | | | | Supernatant (SN) | | Pellet (P) | | |
| | Medium | Solvent | LA (Sa1.1) | LA | % LA-SN/LA-Sa1.1 | LA | % LA-P/LA-Sa1.1 | |
| 15 | pH 4.0 | E | 41428.3 | 1394.3 | 3.4 | 40656.7 | 98.1 | 101.5 |
| 16 | pH 7.0 | A | 56936.9 | 4750.9 | 8.3 | 46955.0 | 82.5 | 90.8 |
| 17 | pH 7.0 | E | 48177.4 | 2316.1 | 4.8 | 41808.2 | 86.8 | 91.6 |
| LA=lipase activity (IU USP);; A= acetone. E=ethanol; S=suspension; SN=supernatant; P=pellet. | | | | | | | | |

Recovery after precipitation is high, almost complete recovery is achieved with the two-steps process.

Table 8

| Sample | Experimental conditions | | Separation (Step a2) | | | | | |
|---|---|---|---|---|---|---|---|---|
| | | | Theor. LA Step1a | Supernatant (SN) | | Pellet (P) | | Yield |
| | Medium | Solvent | | LA | % LA-SN/ theor SL-S1a | LA | % LA-P/theor LA-S1a | |
| 14 | pH 4.0 | A | 61605.4 | 4688.0 | 7.6 | 45021.4 | 73.1 | 80.7 |
| 15 | pH 4.0 | E | 51940.8 | 1394.3 | 2.7 | 40656.7 | 78.3 | 81.0 |
| 16 | pH 7.0 | A | 61421.4 | 4750.9 | 7.7 | 46955.0 | 76.4 | 84.2 |
| 17 | pH 7.0 | E | 51971.9 | 2316.1 | 4.5 | 41808.2 | 80.4 | 84.9 |
| LA=lipase activity (IU USP);; A= acetone. E=ethanol; S=suspension; SN=supernatant; P=pellet; Theor.= theoretical. | | | | | | | | |

The overall process yield ranges from 73.1 to 84.5, which is higher than that obtained with the multi-step process. The yield % is the total lipase activity in the pellet and in supernatant of step a2 with respect to theoretical lipase activity of pancreatin used in step a1.1, which is calculated by factoring specific activity of starting material (i.e., 94.4 IU USP/mg as determined according to compendial USP method) and its initial weight.

Table 9

| Sample | Experimental conditions | | Separation (Step a2) | | EF |
|---|---|---|---|---|---|
| | Medium | Solvent | Supernatant (SN) | Pellet (P) | |
| | | | LSA | LSA | |
| 14 | pH 4.0 | A | 10.1 | 247.4 | 2.6 |
| 15 | pH 4.0 | E | 4.0 | 204.3 | 2.2 |
| 16 | pH 7.0 | A | 10.1 | 276.2 | 2.9 |
| 17 | pH 7.0 | E | 6.7 | 193.6 | 2.1 |
| LSA= lipase specific activity (IU USP/mg); A= acetone. E=ethanol; SN=supernatant; EF= enrichment factor = LSA in pellet step a2 (IU USP/mg)/ theoretical lipase activity in step a1.1 (IU USP/mg). | | | | | |

The results show that the use of the aqueous buffer at pH=7 and acetone in two-steps process provide interesting yield (overall process yield of about 76%) and enrichment factor ranges from 2.1 to 4.2.

**Example 4 (comparative example) Preparation of HA-pancreatin; 0.1 g/mL; two-steps: suspension, precipitation; scale-up (SP= 38: acetone: aqueous solvent=35:65)**

Step a1.1- Suspension: 6.5 g of starting pancrelipase (61,400 U lipase) is dispersed in 45 mL of pH=7.0 buffer solution (10 mM phosphate buffer) at 4°C and stirred (Ultraturrax, 3 cycles) for 1 minute for each cycle, stirrer is washed two times with 10 ml of cold buffer in order to recover the residual pancrelipase.

Step a1.2- Precipitation: 35 mL of acetone is added to the suspension of step a1.1 and the mixture is kept at 4°C for 30 minutes under static condition.

Step a2- Separation: the mixture is centrifuged (15 minutes, 4°C, about 2,700 g) to separate supernatant from pellet, which contains the pancreatic enzymes.

Steps a3- Drying: the pellet is dried according to two different protocols the mean weighted dried pellet is 1.55 g, the lipase activity (LA) is 365,000 IU lipase, and the specific activity (LSA) is 235 IU USP/mg.

Table 10 reports lipase (L), protease (P), and amylase (A) specific activities measured for each material obtained with the two protocols. Lipase activity in the pellets (measured after its suspension in 1 mL of buffer) is measured also before the drying treatment. It amounts to 233.7 IU USP/mg.

Table 10

| Sample | Protocols | LSA | PSA | ASA | P/L ratio | A/L ratio | Water content (%) |
|--------|-----------|-----|-----|-----|-----------|-----------|-------------------|
| 18 | 72 h 6-8° C 0.2 mbar | 234.2 | 226.5 | 149.5 | 0.97 | 0.64 | 7.4 |
| 19 | 72 h 6-8° C 0.2 mbar | 254.0 | 217.7 | 130.7 | 0.86 | 0.51 | 6.5 |
| 20 | 24 h 6-8° C 0.2 mbar | 216.3 | 230.1 | 129.8 | 1.06 | 0.60 | 7.4 |
| Mean | | 234.8 | 224.8 | 136.7 | 0.96 | 0.58 | 7.10 |
| Ds | | 18.9 | 6.4 | 11.1 | | | |
| cv% | | 8% | 3% | 8% | | | |
| LSA= lipase specific activity (IU USP/mg); PSA= protease specific activity (IU USP/mg); ASA = amylase specific activity (IU USP/mg). | | | | | | | |

The analysis shows that drying process itself does not affect LSA and that different protocols leads to materials with same enzymatic activities. The enrichment factor calculated as in previous examples is also always above 2 and is constant among the different protocols, it is: 2.5 (sample 18), 2.7 (sample 19); 2.3 (sample 10), mean value is 2.5. Lipase activity in the pellets, measured before the drying treatment, amounts to 233.7 IU USP/mg (sample 18).

Table 11

| Sample | W starting pan | W pellet | Initial LA | Initial PA | Initial AA | Recovered LA | Recovered PA | Recovered AA | LY | PY | AY |
|---|---|---|---|---|---|---|---|---|---|---|---|
| 18 | 6.5113 | 1.5325 | 614666.7 | 1648661.2 | 2736699.4 | 358911.5 | 347111.25 | 229108.75 | 58 | 21 | 8 |
| 19 | 6.4997 | 1.7084 | 613571.7 | 1645724.0 | 2731823.9 | 369526.92 | 393102.84 | 221750.32 | 60 | 24 | 8 |
| 20 | 6.5137 | 1.3673 | 614893.3 | 1649268.8 | 2737708.1 | 347294.2 | 297661.21 | 178706.11 | 56 | 18 | 7 |
| L= lipase; P= protease; A= amylase; A= activity; Y=yield (%); W=weight (g) measured after drying. | | | | | | | | | | | |

The HPLC profiles of samples obtained with the different protocols are super-imposable. No relevant qualitative difference with the HPLC profile of the starting material was observed.

**Example 5** (comparative example) Preparation of HA-pancreatin; 0.1 g/mL; two-steps: suspension, precipitation; scale-up (SP= 38: ethanol: aqueous solvent (pH=7) = 45: 55, sample 21); (SP= 34: acetone: aqueous solvent (pH=7) = 50: 50 sample 22); (SP= 35: acetone: aqueous solvent (pH=7) = 45: 55, sample 23). The preparation process of Example 4 is here applied on different amount of starting pancrelipase, different volume of buffer solution and different volume of acetone (HS=34 or 35) or volume of ethanol (HS=38). The drying protocol is 24 h, 6-8°C, 0.2 mbar, all other parameters and conditions are the same as in Example 4.

Table 12

| Sample | HS | Pancrelipase (g) | Buffer (mL) | Acetone (mL) | Ethanol (mL) |
|--------|-----|------------------|-------------|--------------|--------------|
| 21 | 38 | 5.5 | 55 | no | 45 |
| 22 | 34 | 5.0 | 50 | 50 | no |
| 23 | 35 | 5.5 | 55 | 45 | no |

Results are reported in Tables 13 and 14.

Table 13

| Sample | LSA | PSA | ASA | P/L ratio | A/L ratio |
|--------|-------|-------|-------|-----------|-----------|
| 21 | 150.2 | - | - | - | - |
| 22 | 123.3 | 351.7 | 436.0 | 2.85 | 3.54 |
| 23 | 158.7 | 388.9 | 241.9 | 2.45 | 1.52 |
| LSA= lipase specific activity (IU USP/mg); PSA= protease specific activity (IU USP/mg); ASA= amylase specific activity (IU USP/mg). | | | | | |

The data shows that drying process itself does not affect LA and that different protocols leads to materials with the same enzymatic activities. The enrichment factor calculated as in previous Examples is: 1.6 (sample 21), 1.3 (sample 22), 1.7 (sample 23).

Table 14 shows the enzymatic activities of the final purified material obtained with the scaled-up process here applied and the enzymatic yield.

| Sample | W starting pan | Wof pellet | Initial LA | Initial PA | Initial AA | Recovered LA | Recovered PA | Recovered AA | LY | PY | AY |
|---|---|---|---|---|---|---|---|---|---|---|---|
| 21 | 5.5197 | 1.6826 | 514988.0 | - | - | 252390.0 | - | - | 49 | - | - |
| 22 | 5.5183 | 2.0255 | 520927.5 | 1397233.6 | 2319341.5 | 321446.85 | 787716.95 | 489968.45 | 62 | 56 | 21 |
| 23 | 4.9986 | 2.7464 | 471867.8 | 1265645.5 | 2100911.6 | 338631.12 | 965908.88 | 1197430.4 | 72 | 76 | 57 |
| L= lipase; P= protease; A= amylase; A= activity; Y=yield (%); W=weight (g) measured after drying. | | | | | | | | | | | |

The HPLC profiles of samples obtained with the different freeze-drying protocols are super-imposable. No relevant qualitative difference with the HPLC profile of the starting material is observed.

Samples 25 and 26 obtained with different protocol show a lower specific LA and higher PA and AA over the sample 24 produced with protocol (HS=38).

**Example 6** Preparation of HA-pancreatin; 0.065 and 0.1 g/mL; single-step; lab scale (SP= 38- acetone: aqueous solvent = 35: 65)

Step a1- Suspension- precipitation: 650 mg (sample 24) or 1000 mg (sample 25) of native pancrelipase are dispersed in 10 mL of a 65:35 mixture of buffer (pH=7 10 mM phosphate) and acetone (65 volumes of buffer and 35 volumes of acetone) under stirring for 30 minutes at 4°C.

Step a2- Separation: the mixture of step a1 is centrifuged (10 min at 10,000 g at 4 °C) to separate supernatant from pellet, which contains the pancreatic enzymes.

Step a3)- Drying: the pellets are dried with a high efficiency pump at 0.2 mbar.

The material is analyzed. The amount of pancrelipase, expressed as lipase activity is measured along the whole process in:

- in supernatant obtained in step a2 (LA in SN, separation, LA-SN);

- in pellets obtained in step a2 and then re-suspended in the starting medium (LA in pellets, precipitation, LA-P);

- the LA of the suspension - precipitation step a1 (LA in suspension; LA-SN) is expressed as theoretical value.

Results are reported in Tables 15 and 16.

Table 15

| Sample | Experimental conditions | | | Separation (Step a2) | | | | |
| --- | --- | --- | --- | --- | --- | --- | --- | --- |
| | Medium | Solvent | Theor. Initial LA Step a1 | Supernatant (SN) | | Pellet (P) | | Yield |
| | | | | LA | % LA-SN/ theor SL-Sa1 | LA | % LA-P/theor LA-Sa1 | |
| 24 | pH 7.0 | A | - | 2834.7 | - | 47807.9 | - | - |
| 25 | pH 7.0 | A | - | 3360.4 | - | 71997.8 | - | - |
| LA=lipase activity (IU USP); A= acetone; E=ethanol; S=suspension; SN=supernatant; P=pellet; Theor.= theoretical. | | | | | | | | |

Table 16

| Sample | Experimental conditions | | Separation (Step a2) | | EF |
| --- | --- | --- | --- | --- | --- |
| | Medium | Solvent | Supernatant (SN) | Pellet (P) | |
| | | | LSA | LSA | |
| 24 | pH 7.0 | A | 5.4 | 237.9 | 2.5 |
| 25 | pH 7.0 | A | 5.7 | 265.7 | 2.8 |
| LA= lipase activity; A= acetone. E=ethanol; SN=supernatant; EF= enrichment factor = LSA in pellet step a2 (IU USP/mg)/ theoretical lipase activity in step a1 (IU USP/mg). | | | | | |

The single-step process provides good yield and higher enhancement factor than two-steps process. It is interesting for industrialization since the execution is easy and straightforward.

**Example 7** Preparation of HA-pancreatin; 0.1 g/mL; single-step; pilot scale (SP= 38-acetone: aqueous solvent = 35: 65)

Step a1- Suspension- precipitation: 10 g of native pancrelipase are dispersed in 100 mL of a solvent mixture of buffer (pH=7, 10 mM phosphate) and acetone (65 volumes of buffer and 35 volumes of acetone) under stirring for 60 minutes at 4°C.

Step a2- Separation: the mixture of step a1 is centrifuged (10 min at 2,700 g at 4°C) to separate supernatant from pellet, which contains the pancreatic enzymes.

Step a3) -Drying, the pellets of sample 26 is poured into a petri dishes, whereas pellet of samples 28 and 29 are kept in the centrifugation tubes and directly dried using a high efficiency pump at 0.2 mbar.

The material is analyzed. The amount of pancrelipase, expressed as lipase activity is measured along the whole process in:

- in supernatant obtained in step a2 (LA in SN, separation, LA-SN);

- in pellets obtained in step a2 and then re-suspended in the starting medium (LA in pellets, separation, LA-P);

- the LA of the suspension - precipitation step a1 (LA in suspension; LA-SN) is expressed as theoretical value.

Results are reported in Tables 17 and 18.

Table 17

| Sample | LSA | PSA | ASA | P/L ratio | A/L ratio |
|---|---|---|---|---|---|
| 26 | 207.8 | 249.4 | 155.20 | 1.20 | 0.75 |
| 27 | 222.7 | 249.2 | 158.90 | 1.12 | 0.71 |
| 28 | 219.1 | 285.0 | 167.50 | 1.30 | 0.76 |

Good reproducibility in term of lipase, protease, and amylase activity is obtained.

Table 18

| Sample | W starting pan | W of PELLET | LY | PY | AY |
|---|---|---|---|---|---|
| 26 | 10.02 | 3.94 | 87 | 39 | 15 |
| 27 | 10.01 | 4.22 | 100 | 42 | 16 |
| 28 | 10.02 | 4.28 | 99 | 48 | 17 |
| L= lipase; P= protease; A= amylase; A= activity; Y=yield (%); W=weight (g) measured after drying. | | | | | |

Very high lipase yield is obtained, also enrichment factor is high (above 2): it is: 2.2 (Sample 26), 2.4 (Sample 27), 2.3 (Sample 28).

The single-step process provides good yields. 4.2 gr of HA-pancreatin is obtained (42% of the starting pancrelipase), total units of lipase is 940,000 IU USP (99-100% of the initial LA). The obtained HA-pancreatin has specific activity of 221 IU USP/mg.

**Example 8** Preparation of HA-pancreatin; 0.1 g/mL; multi-steps: suspension, precipitation, separation; comparative example (SP= 38- acetone: water = 35: 65)

Step a1.1- Suspension: pancrelipase is dispersed in aqueous solvent (distilled water at concentration of 0.045 mg/mL at 4°C (sample 29), for about 30 minutes under stirring.

Step a1.2- Precipitation: 80 mL of the solvent mixture (acetone: water = 35:45) is added to 20 mL of suspension of step a1.1 (to obtain SP=38 $(MPa)^{0.5}$ in the final solvent mixture); the mixture is kept at 25°C for 60 minutes under static condition.

Step a2- Separation: the mixture is centrifuged (10 min at 3,000 g at 25°C) to separate supernatant from pellet, which contains the pancreatic enzymes.

The materials of the different steps are analyzed. The amount of pancrelipase, expressed as lipase activity is measured along the whole process:

- in suspension of step a1.1 (LA in suspension; LA-Sa1.1);

- in supernatant obtained in step a2 (LA in SN, precipitation, LA-SN);

- in pellets obtained in step a2 and then re-suspended in the starting distilled water (LA in pellets, precipitation, LA-P).

Results are reported in Tables 19-20. Results obtained with two-steps and one-step methods in previous Examples are also reported here for direct comparison purposes.

Table 19

| Sample | Experimental conditions | | | Separation (Step a2) | | | | |
| | Medium | Solvent | Theor. Initial LA Step a1.1 | Supernatant (SN) | | Pellet (P) | | Yield |
| | | | | LA | % LA-SN/theor SL-Sa1.1 | LA | % LA-P/theor LA-Sa1.1 | |
| 16 | pH 7.0 | A | 56936.9 | 4750.9 | 8.3 | 46955.0 | 82.5 | 90.8 |
| 18 | pH 7.0 | A | - | 2834.7 | - | 47807.9 | - | - |
| 19 | pH 7.0 | A | - | 3360.4 | - | 71997.8 | - | - |
| 29 | Water | A | 66313.9 | 7928.8 | 12.0 | 24082.9 | 36.3 | 48.3 |
| LA=lipase activity (IU USP); A= acetone, E=ethanol; S=suspension; SN=supernatant; P=pellet; Theor.= theoretical. | | | | | | | | |

Table 20

| Sample | Experimental conditions | | Precipitation (Step a2) | | EF |
| | Medium | Solvent | Supernatant (SN) | Pellet (P) | |
| | | | LSA | LSA | |
| 16 | pH 7.0 | A | 10.1 | 276.2 | 2.9 |
| 18 | pH 7.1 | A | 5.4 | 237.9 | 2.5 |
| 19 | pH 7.0 | A | 5.7 | 265.7 | 2.8 |
| 29 | Water | A | 9.7 | 27.1 | 0,3 |
| LSA= lipase specific activity (IU USP/mg); A= acetone. E=ethanol; SN=supernatant; EF= enrichment factor = LSA in pellet step a2 (IU USP/mg)/ theoretical lipase activity in step a1 (IU USP/mg). | | | | | |

This prior art process provides poor yield, enzymatic inactivation is pronounced and consequently no lipase enrichment is obtained.

**Example 9** Characterization of purified HA-pancreatin (Sample20)

The HA material is tested for its digestion capability and compared with starting pancrelipase. 20 mg of HA-pancreatin and 50 mg of starting pancrelipase (corresponding to 2300 IU USP lipase) are suspended each in 1 mL of deionised water and the added to 50 mL of enteral formula Peptamen® Junior 1.0 at 37°C and stirred at 100 rpm for 60, 120 and 240 minutes. Digestion tests are carried out after 1, 2 and 4 hours. The test was repeated 6 times for each pancrelipase sample.

Lipidic nutrients are monitored by measuring the digestion of triolein (decrease of triolein peak). The total protein digestion is monitored by the Bradford method. Amyliolitic process is monitored by measuring the formation of short chain sugars. Digestion extent difference is obtained by comparing the digestion performance of native and HA materials after 4 hours digestion.

Table 21

| Sample | W | Lipase | Protease | Amylase |
|---|---|---|---|---|
| N | 50 | 4,720 | 12,660 | 21,015 |
| H | 20 | 4,142 | 4,354 | 2,614 |
| Activity difference (%) | | -12 | -66 | -88 |
| Digestion difference (%) | | -10% | 6 | -33 |
| H= HA-pancreatin; N= native pancreatin; W= weight (mg) | | | | |

Activity difference is calculated with the formula:

$$\text{(activity of native pancreatin} - \text{activity of HA-pancreatin)} / \text{activity of native pancrelipase}$$

Digestion difference is calculated by the following formula:

$$\text{(% of digestion of native pancreatin} - \text{% digestion of HA-pancreatin)}$$

This *in vitro* test allows to analyze the lipase, protein and carbohydrate digestion of HA-pancreatin. Lipase digestion is extremely sensitive to difference in enzymatic activities present in the reaction vessels hence to 10 % of difference in the activity corresponds to 10 % of difference in digestion amount. Digestion kinetics shows similar trends. These results suggest that native and HA-pancreatin have similar lipidic digestion pattern.

Proteins digestion profiles are almost superimposable suggesting that protease activity of HA-pancreatin sustains the digestion at the same level of the native pancrelipase. A difference of about 60% of activity for protease does not produce any effects on protein digestion extent.

Carbohydrates digestion profiles are different since maltose production is lower in HA-pancreatin. The comparison of the difference in amylase activity and of the amount of digested products shows that in the HA-pancreatin also the amylolitic occurs.

**Claims**

1.  A process for the preparation of HA-pancreatin having specific lipase activity of at least about 120 USP IU/mg, wherein the pancreatin is of mammalian origin and comprises lipase, protease and amylase,

    the process comprising treating the mammalian pancreatin with a solvent having Hildebrand solubility parameter comprised between 28 and 45 $(MPa)^{0.5}$
    wherein said solvent is a mixture of at least one organic solvent and aqueous solvent, , and wherein the process comprises the following steps:

    a1) suspending pancreatin in the mixture of at least one organic solvent and aqueous solvent; or

a1.1) suspending pancreatin in aqueous solvent under stirring;
a1.2) adding to the suspension of step a.1.1 the one organic solvent, and

a2) separating the insoluble portion from the soluble portion of the mixture of step a1; and
a3) drying the insoluble portion obtained in step a2; wherein said process steps are carried out at about 4°C;

and recovering the HA-pancreatin comprising lipase, protease and amylase, and having a specific lipase activity of at least about 120 USP IU/mg.

2. The process of claim 1, wherein the mixture of solvents has Hildebrand solubility parameter comprised between 28 and 38 $(MPa)^{0.5}$; 28 and 34 $(MPa)^{0.5}$; 34 and 38 $(MPa)^{0.5}$; or 38 and 45 $(MPa)^{0.5}$.

3. The process of claim 1, wherein the mixture of solvents has Hildebrand solubility parameter comprised between 34 and 45 $(MPa)^{0.5}$

4. The process of any one of claims 1 to 3, for the preparation of pancreatin having specific lipase activity of at least about 150 USP IU/mg, 200 USP IU/mg, or 250 USP IU/mg.

5. The process of any one of claims 1 to 4, wherein step a1.1 is carried out for about 10 to 30 minutes.

6. The process of any one of claims 1 to 5, wherein the pancreatin of step a1.1 or a1 is in amount comprised between 0.05 and 0.3 mg/mL.

7. The process of any one of claims 1 to 6, wherein the mixture of solvents has Hildebrand solubility parameter of 38 $(MPa)^{0.5}$.

8. The process of any one of claims 1 to 7, wherein the organic solvent is chosen from the group of: n-pentane, n-hexane, n-heptane, diethylether, cyclohexane, carbon tetrachloride, ethylacetate, tetrahydrofuran, chloroform, trichloroethylene, acetone, dimethylformamide, n-propanol, isopropanol, ethanol, dimethylsulfoxide butylalcohol, methanol, acetonitrile, dioxane, and methylenchloride.

9. The process of claim 8, wherein the organic solvent is chosen from the group of acetone and ethanol.

10. The process of any one of claims 1 to 9, wherein the aqueous solvent is buffer solution, wherein the buffer solution optionally has pH=7 or pH=4.

11. The process of any one of claims 1 to 10, wherein the organic solvent is acetone and the aqueous solvent is buffer solution with pH=7.

12. The process of claim 1, wherein the mixture of solvents has Hildebrand solubility parameter of 38 $(MPa)^{0.5}$ and wherein the mixture of solvents is a mixture of acetone and buffer solution with pH= 7 and the pancreatin in step a1 or step a1.1 is in concentration of 0.1 mg/mL.

13. The process of any one of claims 1 to 12 comprising the step of microbial and /or viral load reduction, wherein the bacterial and/or viral load reduction is optionally carried out by filtration, heating, ionizing radiation, high pressure or by alkylation.

**Patentansprüche**

1. Verfahren zur Herstellung von HA-Pankreatin, das eine spezifische Lipase-Aktivität von zumindest etwa 120 USP IU/mg aufweist, wobei das Pankreatin von einem Säugetier stammt und Lipase, Protease und Amylase umfasst, wobei das Verfahren das Behandeln des Säugetier-Pankreatins mit einem Lösungsmittel, das Hildebrand-Löslichkeitsparameter aufweist, der zwischen 28 und 45 $(MPa)^{0,5}$ liegt, umfasst und wobei das Verfahren die folgenden Schritte umfasst:

a1) das Suspendieren von Pankreatin in dem Gemisch aus zumindest einem organischen Lösungsmittel und wässrigem Lösungsmittel oder

a1.1) das Suspendieren von Pankreatin in wässrigem Lösungsmittel unter Rühren,

a1.2) das Zusetzen des einen organischen Lösungsmittels zur Suspension aus Schritt a1.1 und

a2) das Trennen des unlöslichen Teils vom löslichen Teil des Gemischs aus Schritt a1; und

a3) das Trocknen des unlöslichen Teils, der in Schritt a2 erhalten wurde, wobei die Verfahrensschritte bei etwa 4 °C durchgeführt werden,

und das Gewinnen des HA-Pankreatins, umfassend Lipase, Protease und Amylase, das eine spezifische Lipase-Aktivität von zumindest etwa 120 USP IU/mg aufweist.

2. Verfahren nach Anspruch 1, wobei das Gemisch an Lösungsmitteln Hildebrand-Löslichkeitsparameter aufweist, der zwischen 28 und 38 $(MPa)^{0,5}$, 28 und 34 $(MPa)^{0,5}$, 34 und 38 $(MPa)^{0,5}$ oder 38 und 45 $(MPa)^{0,5}$ liegt.

3. Verfahren nach Anspruch 1, wobei das Gemisch an Lösungsmitteln Hildebrand-Löslichkeitsparameter aufweist, der zwischen 34 und 45 $(MPa)^{0,5}$ liegt.

4. Verfahren nach einem der Ansprüche 1 bis 3 zur Herstellung von Pankreatin, das eine spezifische Lipase-Aktivität von zumindest etwa 150 USP IU/mg, 200 USP IU/mg oder 250 USP IU/mg aufweist.

5. Verfahren nach einem der Ansprüche 1 bis 4, wobei Schritt a1.1 etwa 10 bis 30 min lang durchgeführt wird.

6. Verfahren nach einem der Ansprüche 1 bis 5, wobei das Pankreatin aus Schritt a1.1 oder a1 in einer Menge zwischen etwa 0,05 und 0,3 mg/ml umfasst ist.

7. Verfahren nach einem der Ansprüche 1 bis 6, wobei das Gemisch aus Lösungsmitteln Hildebrand-Löslichkeitsparameter von 38 $(MPa)^{0,5}$ aufweist.

8. Verfahren nach einem der Ansprüche 1 bis 7, wobei das organische Lösungsmittel aus der folgenden Gruppe ausgewählt ist: n-Pentan, n-Hexan, n-Heptan, Diethylether, Cyclohexan, Kohlenstofftetrachlorid, Ethylacetat, Tetrahydrofuran, Chloroform, Trichlorethylen, Aceton, Dimethylformamid, n-Propanol, Isopropanol, Ethanol, Dimethylsulfoxid, Butylalkohol, Methanol, Acetonitril, Dioxan und Methylenchlorid.

9. Verfahren nach Anspruch 8, wobei das organische Lösungsmittel aus der aus Aceton und Ethanol bestehenden Gruppe ausgewählt ist.

10. Verfahren nach einem der Ansprüche 1 bis 9, wobei das wässrige Lösungsmittel eine Pufferlösung ist, wobei die Pufferlösung gegebenenfalls einen pH = 7 oder einen pH = 4 aufweist.

11. Verfahren nach einem der Ansprüche 1 bis 10, wobei das organische Lösungsmittel Aceton ist und das wässrige Lösungsmittel eine Pufferlösung mit pH = 7 ist.

12. Verfahren nach Anspruch 1, wobei das Gemisch aus Lösungsmitteln Hildebrand-Löslichkeitsparameter von 38 $(MPa)^{0,5}$ aufweist und wobei das Gemisch aus Lösungsmitteln ein Gemisch aus Aceton und Pufferlösung mit pH = 7 ist und das Pankreatin in Schritt a1 oder Schritt a1.1 eine Konzentration von 0,1 mg/ml aufweist.

13. Verfahren nach einem der Ansprüche 1 bis 12, umfassend einen Mikroben- und/oder Viruslastreduktionsschritt, wobei die Bakterien- und/oder Virenlastreduktion gegebenenfalls durch Filtration, Erhitzen, ionisierende Bestrahlung, Hochdruck oder Alkylierung durchgeführt wird.

**Revendications**

1. Procédé pour la préparation de pancréatine-HA possédant une activité lipase spécifique d'au moins environ 120 USP IU/mg, dans lequel la pancréatine est d'origine mammifère et comprend une lipase, protéase et amylase,

le procédé comprenant le traitement de la pancréatine de mammifère avec un solvant possédant un paramètre de solubilité de Hildebrand compris entre 28 et 45 $(MPa)^{0,5}$,
dans lequel ledit solvant est un mélange d'au moins un solvant organique et solvant aqueux, et où le procédé comprend les étapes suivantes consistant à :

a1) mettre en suspension la pancréatine dans le mélange d'au moins un solvant organique et solvant aqueux ; ou

a1.1) mettre en suspension la pancréatine dans un solvant aqueux sous agitation;
a1.2) ajouter, à la suspension de l'étape a1.1, le un solvant organique ; et

a2) séparer la partie insoluble de la partie soluble du mélange de l'étape a1; et
a3) sécher la partie insoluble obtenue à l'étape a2 ; où lesdites étapes du procédé sont réalisées à environ 4 °C;

et la récupération de la pancréatine-HA comprenant une lipase, protéase et amylase, et possédant une activité lipase spécifique d'au moins environ 120 USP IU/mg.

2. Procédé selon la revendication 1, dans lequel le mélange de solvants possède un paramètre de solubilité de Hildebrand compris entre 28 et 38 $(MPa)^{0,5}$; 28 et 34 $(MPa)^{0,5}$ ; 34 et 38 $(MPa)^{0,5}$ ; ou 38 et 45 $(MPa)^{0,5}$.

3. Procédé selon la revendication 1, dans lequel le mélange de solvants possède un paramètre de solubilité de Hildebrand compris entre 34 et 45 $(MPa)^{0,5}$.

4. Procédé selon l'une quelconque des revendications 1 à 3, pour la préparation d'une pancréatine possédant une activité lipase spécifique d'au moins environ 150 USP IU/mg, 200 USP IU/mg, ou 250 USP IU/mg.

5. Procédé selon l'une quelconque des revendications 1 à 4, dans lequel l'étape a1.1 est réalisée pendant environ 10 à 30 minutes.

6. Procédé selon l'une quelconque des revendications 1 à 5, dans lequel la pancréatine de l'étape a1.1 ou a1 est présente en une quantité comprise entre 0,05 et 0,3 mg/ml.

7. Procédé selon l'une quelconque des revendications 1 à 6, dans lequel le mélange de solvants possède un paramètre de solubilité de Hildebrand de 38 $(MPa)^{0,5}$.

8. Procédé selon l'une quelconque des revendications 1 à 7, dans lequel le solvant organique est choisi dans le groupe suivant : n-pentane, n-hexane, n-heptane, éther diéthylique, cyclohexane, tétrachlorure de carbone, acétate d'éthyle, tétrahydrofurane, chloroforme, trichloroéthylène, acétone, diméthylformamide, n-propanol, isopropanol, éthanol, diméthylsulfoxyde, alcool butylique, méthanol, acétonitrile, dioxane, et chlorure de méthylène.

9. Procédé selon la revendication 8, dans lequel le solvant organique est choisi dans le groupe de l'acétone et l'éthanol.

10. Procédé selon l'une quelconque des revendications 1 à 9, dans lequel le solvant aqueux est une solution tampon, où la solution tampon possède facultativement un pH = 7 ou un pH = 4.

11. Procédé selon l'une quelconque des revendications 1 à 10, dans lequel le solvant organique est l'acétone et le solvant aqueux est une solution tampon possédant un pH = 7.

12. Procédé selon la revendication 1, dans lequel le mélange de solvants possède un paramètre de solubilité de Hildebrand de 38 $(MPa)^{0,5}$ et dans lequel le mélange de solvants est un mélange d'acétone et d'une solution tampon possédant un pH = 7 et la pancréatine à l'étape a1 ou l'étape al.l est présente en une concentration de 0,1 mg/ml.

13. Procédé selon l'une quelconque des revendications 1 à 12, comprenant l'étape de réduction de la charge microbienne et/ou virale, où la réduction de la charge microbienne et/ou virale est facultativement réalisée par filtration, chauffage, rayonnement ionisant, haute pression ou par alkylation.

**EP 3 024 479 B2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6051220 A
- US 20040057944 A
- US 20010046493 A
- WO 2006044529 A

**Non-patent literature cited in the description**

- **HWANG.** *Ind. Eng. Chem. Res.,* 2007, vol. 46, 4289
- **BARTON.** Handbook of Solubility Pa-rameters. CRC Press, 1983